(19)

Europäisches
Patentamt
European
Patent Office
Office européen
des brevets

(11) **EP 1 576 965 B1**

(12) **EUROPEAN PATENT SPECIFICATION**

(45) Date of publication and mention
of the grant of the patent:
**14.10.2009 Bulletin 2009/42**

(21) Application number: **03767380.3**

(22) Date of filing: **22.12.2003**

(51) Int Cl.:
**A61K 39/00** *(2006.01)*    **A61K 39/395** *(2006.01)*

(86) International application number:
**PCT/CU2003/000019**

(87) International publication number:
**WO 2004/058297 (15.07.2004 Gazette 2004/29)**

(54) **COMBINATIONS OF GROWTH- AND HORMONE-REGULATING FACTORS FOR THE TREATMENT OF NEOPLASIA**

KOMBINATIONEN WACHSTUMS- UND HORMONSTEUERNDER FAKTOREN FÜR DIE BEHANDLUNG VON NEOPLASIE

COMBINAISONS DE FACTEURS REGULATEURS DE CROISSANCE ET D'HORMONES DANS LE TRAITEMENT DES NEOPLASIES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.12.2002 CU 33802**

(43) Date of publication of application:
**21.09.2005 Bulletin 2005/38**

(73) Proprietor: **CENTRO DE INGENIERIA GENETICA Y BIOTECNOLOGIA (CIGB)**
**Ciudad de la Habana 10600 (CU)**

(72) Inventors:
• **BOVER FUENTES, Eddy**
**70 800 Camagüey (CU)**
• **BASULTO BAKER, R.,**
**Sociedad Patriotica, 6A entre**
**70 300 Camagüey (CU)**
• **PIMENTEL VAZQUEZ, Eulogio**
**70 800 Camagüey (CU)**
• **JUNCO BARRANCO, Jesus**
**70 800 Camagüey (CU)**
• **FUENTES AGUILAR, Franklin**
**70 800 Camagüey (CU)**
• **ARTEAGA MORE , Niurka**
**70 800 Camagüey (CU)**
• **CALZADA AGUILERA, Lesvia**
**70 800 Camagüey (CU)**
• **HERNANDEZ DOMINGUEZ, Héctor**
**70 800 Camagüey (CU)**
• **Lopez Saez, Yovisleidys**
**Reparto Julio A. Mella,**
**70 600 Camagüey (CU)**
• **GUILLEN NIETO, Gerardo, Enrique**
**10400 Ciudad de La Habana (CU)**
• **Chinea Santiago, Glay**
**Ciudad Habana 12 100, Cuba (CU)**

(74) Representative: **Winckels, Johannes Hubertus F. et al**
**Vereenigde**
**Johan de Wittlaan 7**
**2517 JR Den Haag (NL)**

(56) References cited:
**WO-A-02/45738**    **WO-A-94/10202**
**WO-A-98/27111**    **GB-A- 2 228 262**
**US-A- 5 894 018**

• **CAMBY I ET AL: "CHARACTERIZATION OF THE INFLUENCE OF ANTI-HORMONE AND/OR ANTI-GROWTH FACTOR NEUTRALIZING ANTIBODIES ON CELL CLONE ARCHITECTURE AND THE GROWTH OF HUMAN NEOPLASTIC ASTROCYTIC CELL LINES" JOURNAL OF NEURO-ONCOLOGY, KLUWER, BOSTON, US, vol. 20, no. 1, 1994, pages 67-80, XP000618456 ISSN: 0167-594X**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the invention**

This invention is mainly related to the field of immunology, endocrinology and oncology, and in particular to pharmaceutical compositions comprising a combination of growth regulating factors (EGF,TGF, VEGF), and sexual hormones, and / or those involved in the sexual hormones release cascade or reproduction hormones, which cause a combined auto-immune response for the treatment of neoplasia.

**Prior of the Art**

The Gonadotropin-Releasing Hormone (GnRH), also known as Luteinizing Hormone Releasing Hormone (LHRH), is a hypothalamic peptidic hormone responsible for the release of Luteinizing Hormone (LH) and Follicle Stimulating Hormone (FSH) of the anterior pituitary.

Along with the GnRH produced by the hypothalamic system, there are evidences of GnRH production at other brain sites (Jennes L, Conn P.M. "Gonodatripin-releasing hormone and its receptors in the rat brain". Front Neuroendocrinol. 1994, vol. 15, pp. 51-77), as well as in rat ovary granulose cells (Peng C., Fan N.C., Ligier M., Vaananen J., Leung P.C. "Expression and regulation of gonadotropin-releasing hormone (GnRH) and GnRH receptor messenger ribonucleic acids in human granulosa-luteal cells". Endocrinology 1994, vol. 135, pp. 1740-1746), in testicle cells (Di Matteo L., Vallarino M., Pierantoni R. "Localization of GnRH molecular forms in the brain, pituitary and testis of the frog, Rana esculenta". J. Exp. Zool. 1996, vol. 247, pp 33-40), in human placenta. (Gohar J., Mazor M., Lieberman J.R. "GnRH in pregnancy" Arch. Gynecol. Obstet. 1996, vol 259, pp 1-6), in the immune system (Jacobson J. D., Crofford L.J., Sun L., Wilder R. L. "Cyclical expression of GnRH and GnRH receptor mRNA in lymphoid organs". Neuroendocrinology 1998, vol. 67, pp. 117-125), and pituitary gland (Bauer T.W., Moriarty C.M., Childs G. V. "Studies of immunoreactive gonadotropin releasing hormone (GnRH) in the rat anterior pituitary". J. Histochem. Cytochem, 1981, vol 29, pp 1171-1178).

Gonadectomy is a well-known therapeutic procedure necessary for the treatment of tumors depending on gonadal steroids. GnRH analogues can exert their anti-tumor activity not only through chemical castration, but also by direct effect on tumor cells (Couillard S., Labrie C., Belanger A., Candas B., Pouliot F., Labrie F. "Effect of dehydroepiandrosterone and anti-androgen EM-800 on growth of human ZR-75-1 breast cancer xenografts". J. Nat. Cancer Inst. 1998, May 20, pp. 772-778; Kolle S. et al. "Expression of growth hormone receptor in human prostatic carcinoma and hyperplasia". Int. J. Oncol. 1999, vol. 14, No. 5, pp. 911-916).

Likewise, it has been reported that a GnRH antagonist (MZ-4-71) can suppress the growth of androgen independent prostate cancer cell lines PC-3, DU-145 and Dunning AT-1 (A Jungwirth et al. "Inhibition of in vivo proliferation of androgen-independent prostate cancers by an antagonist of growth hormone-releasing hormone". British Journal of Cancer 1997, vol. 75, No. 11, pp. 1585-1592).

Dunning cell line R3327-G has been widely used for different studies generally associated to the treatment of prostate tumors, as a well established method nowadays. The EGF receptor has been found in prostate sensitive to androgens tumors models Dunning R3327 (Damber J.E., Bergh B., Gafvels M. "Epidermal growth factor receptor content in rat prostatic adenocarcinoma: effects of endocrine treatment". Urol. Res. 1995, vol 23, No. 2, pp. 119-25). It has also been suggested that in subline Dunning R3327-G, the expression of EGF receptor is coordinately under androgenic control (Coordinate loss of growth factors following castration of rats carrying the Dunning R3327 G prostatic tumor" Clin Physiol Biochem, 1992, vol. 9, No. 2, pp. 47-50.)

Epidermal Growth Factor (EGF) is a 53 amino acid polypeptide, with an approximate molecular weight of 6045 Da, which stimulates epithelial and mesenchymal cell proliferation *in vitro* and *in vivo* (Cohen S., Carpenter G., "Human Epidermal Growth Factor: Isolation and chemical and biological properties" PNAS USA, 1975, vol. 72 pp.1317). EGF action is exerted through specific receptors at the cell's membrane. EGF was isolated and purified from murine submaxillar glands for the first time (Cohen S. J. Biol. Chem. 1962, vol.237, No. 1, pp. 555). Later a similar molecule was obtained from the human urine (Cohen S. "Human Epidermal Growth Factor: Isolation and Chemical and Biological Protperties", PNAS USA 1975, vol 72, pp. 1317).

The bio-regulatory action of EGF is made through a membrane receptor (EGF-R), a glycoprotein of about 170 KDa, which gene has been cloned and sequenced. The intracellular domain of the receptor is associated to a specific tyrosine kinase protein activity, with a structural homology to oncogene v-erb-B that shows certain relation to malignant transformation processes. (Helding C.H. Cell, 1984, vol. 37, pp. 9-20).

The presence of EGF-R in tumor cells supports reserved predictions regarding human breast cancer. Approximately 40% of the breast tumors show high-affinity specific binding sites for the EGF. (Rios M.A., et al. "Receptors for Epidermal Growth Factor and Estrogen Predictors of Relapse in Patients with Mammary Carcinoma" Anticancer Research 1998, vol. 8, pp. 173-176). There is also an inverse correlation with the presence of estrogen receptors pointing to EGF-R as a differentiation marker, or indicator for the potential proliferation capacity of malignant cells. (Perez R., Pascual M.R., Macias A., Lage A. Breast Cancer Research and Treatment 1984, vol.4. pp 189-193).

Previous studies developed in Ehrlich ascitic tumor model in Balb/c mice (Lombardero J., et al. Neoplasma 1987, vol. 33 pp.4) proved the *in vivo* inhibitory effect of EGF, suggesting the possibility of considering this molecule as a biological response transformer.

A vaccine composition containing autologous EGF coupled to a carrier protein that inhibits EGF dependent tumor growth with immune effect, without collateral effects, has been developed (US 5894018: Vaccine composition comprising autologous epidermal growth factor or fragment or derivative, thereof having anti-tumor activity and used thereof in the therapy of malignant diseases).

In previous studies there has been reported that the Dunning tumor express high levels of mRNA for vascular endothelial growth factor (VEGF) and its receptors, compared to ventral prostate ("Expression of vascular endothelial growth factor and its receptors in the ventral prostate and Dunning R3327 PAP adenocarcinoma before and after castration", Prostate 1998, vol. 36, No. 2, pp. 71-79). Assays in animal models have shown that androgenic deprivation may lead to vascular regression and that VEGF may be regulated by androgens. In human prostate cancer, VEGF constitutive production by the glandular epithelium was suppressed because of androgenic ablation therapy. The loss of VEGF led to selective apoptosis of endothelial cells in vessels deprived of periendothelial cells (Laura E. et al.: "Selective ablation of immature blood vessels in established human tumors follows vascular endothelial growth factor withdrawal" J. Clin. Invest. 1999, vol. 103, No.2, pp. 159-165).

The VEGF is a specific angiogenic and vasculogenic mitogen of endothelial cells, and plays a role in pathogenic vascularization, which is associated to a number of clinical pathologies including cancer and rheumatoid arthritis. VEGF is a glycosilated, disulfide-linked homodimer, and is expressed in different isoforms (VEGF 121, VEGF 165, VEGF 189 and VEGF 206) with 121-206 residues in humans (Yves A. Muller, et al. "The crystal structure of vascular endothelial growth factor (VEGF) refined to 1.93 A resolution: Multiple copy flexibility and receptor binding" Structure, 1997, vol. 5, No. 10 pp. 1325-1338.)

In general terms, tumor cells show a dramatically reduced dependency on exogen growth signals, and are able to generate many of their own growth signals. This signal independence derived in an enormous manner, damages a critically important homeostatic behavior, which normally operates to ensure appropriate behavior of several types of cells within a tissue.

To reach growth signal autonomy, the cells have created mechanisms which alter extra cellular growth signals, from transcellular translators of these signals into action (Douglas H. and Robert A. W. "The Hallmarks of Cancer (Review)" Cell 2000, vol. 100, pp. 57-70). While the majority of the growth factors are produced by a cell type to stimulate the proliferation of others (process of heterotypical signaling), a large number of cancer cells take the ability of synthesizing growth factors to which they respond by creating a positive feedback link (autocrine stimulation).

The receptors for certain growth factors, which usually perform tyrosine-kinase activity in their cytoplasmic domains, are over expressed in many kinds of cancer cells and as a consequence they develop a hyper response to normal concentrations of growth factors. Overexpression of growth factor receptors may also elicit ligand independent signaling. Independent ligand signaling may be reached, as well, by receptors' structural alterations (the EGF receptor may lose part of its cytoplasmic domain and signalize constitutively).

SOS-Ras-Raf-MAPK cascade plays the key role in signaling due to the action of growth factors. 25% of human tumors have problems in the regulation of Ras protein expression, although the growth signaling routes are altered in all human tumors (almost half of human colon carcinomas carry mutated ras oncogenes, and the rest is thought to be defective in other signaling route components).

Normal cells, like fibroblasts and endothelial cells may play a key role in the proliferation of tumor cells. In a normal tissue, the cells are encouraged to grow through their neighbors' signals (paracrines), or systemic signals (endocrines). Therefore, to explain tumor cell proliferation, heterotrophic signaling among the several types of cells inside the tumor, should be considered as important as the above-mentioned autonomous mechanisms. In this sense, oxygen and other nutrients supplied by the vasculature are essential for these functions, as well as tumor cell survival.

The ability to induce sustained angiogenesis seems to be acquired in a discrete step (or steps) during tumor development, via an "angiogenic switch" from vascular quiescence. Neovascularization is a pre-requisite for clonal expansion associated with the formation of macroscopic tumors.

The mytogenic effect of growth factors in cells lines can be fought back by GnRH analogues, which shows GnRH interaction with the signal transduction mytogenic route. This hypothesis was demonstrated by tyrosine kinase activity inhibition, induced by growth factors in human tumor cells from ovary and endometrium by GnRH agonists, which is partially due to the activation of GnRH induced phosphotyrosine phosphatase.

The treatment with GnRH analogues has been associated to a dramatic decrease of growth factors receptors (EGF, insulin like growth factor 1 (IGF-1)), on tumor cells membrane, and a sharp increase in mRNA levels for EGF-R in tumors. Additionally, anti-proliferative activity and changes in receptor expression for estrogens and androgens in certain tumor lines, have been reported.

Advances in cancer research for over a quarter of a century, have favored the accumulation of doubtless evidence that supports tumorogenesis as a malignant dynamic process with multiple phases. The broad catalogue of malignant cell genotypes is a manifestation of essential alterations in cell physiology. These transformations collectively drive tissue malignant growth in different types of tumors.

Hence, an important problem to cancer therapy yet unsolved, is achieving modulation of active or passive immunoresponse.

**Summary of the invention.**

This invention is the solution to the previously described problem, using a new pharmaceutical combination that comprises growth regulating factors (EGF, or VEGF) and sexual hormones, and / or those involved in the sexual hormone release cascade, or those involved in reproduction (GnRH). This combination is useful to the treatment of neoplasia, and depending on the circumstances the active ingredients of the combination can be applied simultaneously, separately or sequentially.

In pre-clinical trials, the generation of combined immune response to the above mentioned growth factors and hormones, has allowed for better results than those observed when the immune response to such factors and hormones is generated independently. These results provide evidences that this approximation constitutes a more effective way for the patients' treatment with neoplasias of different origins, since there is promoted the anti-tumor response expressed as reduction of the tumour mass and the increase of the survival time.

More particularly, this invention refers to pharmaceutical combinations for the treatment of neoplasia, for simultaneous, separate or sequential administration, comprising a compound A and a compound B; where A and B are selected from the group of molecules consisting in:

A:

a.1. GnRH, or its analogues, or anti-GnRH antibodies, or GnRH receptor (GnRH-R), or its mutated variants, or derivative peptides, or anti-GnRH antibodies coupled or not to an immunopotentiating carrier protein.
a.2 Natural or recombinant gonadotropins, or their analogues, or mutated variants coupled or not to an immunopotentiating carrier protein, anti-gonadotropin antibodies, their Fabs, scFV fragments, humanized or not.
a.3. Gonadotropin receptors or their mutated variants, or derivative peptides, coupled or not to an immunopotentiating carrier protein.
a.4. anti-Gonadotropin receptor antibodies, their Fabs, scFV fragments, humanized or not.

B.

b.1. Natural or recombinant EGF, or its mutated variants, or derivative peptides or EGF mimetic peptides, or EGF analogues, coupled or not to an immunopotentiating carrier protein.
b.2 Anti-EGF antibodies, their Fabs, scFV fragments, humanized or not.
b.3 EGF receptor (EGF-R), or its mutated variants, or derivative peptides coupled or not to an immunopotentiating carrier protein.
b.4 Anti EGF receptor antibodies, their Fabs, scFV fragments, humanized or not.
b.5 Natural or recombinant VEGF or its mutated variants, or derivative peptides, or VEGF mimetic peptide, or VEGF analogues, coupled or not to an immunopotentiating carrier protein.
b.6 Anti-VEGF antibodies, their Fabs, scFV fragments, humanized or not.
b.7 VEGF receptors, or their mutated variants, or derivative peptides from VEGF receptors, coupled or not to an immunopotentiating carrier protein.
b.8 Anti VEGF receptor antibodies, their Fabs, scFV fragments, humanized or not.

In a preferred formulation, the pharmaceutical combinations that include molecules in the A or B pools, are coupled to the immunopotentiating carrier protein by conjugation, or the formation of chimeric proteins. More particularly, inside the A molecules, GnRH analogue peptide with a sequence of pGlu-His-Trp-Ser-Tyr-Pro-Leu-Arg-Pro-Gly.

Another invention realization of the selected immunopotentiating carrier protein may be one of the Neisseria meningitides P1 or P64 outer membrane proteins, or a Teatanic Toxoid (TT) T helper epitope.

Likewise, this invention refers to a pharmaceutical combination where the conjugated or chimeric protein is one of the following variants:

(b) GnRH bound to a carrier protein and to EGF.
(c) GnRH bound to a carrier protein and to VEGF.

This invention provides a method to generate a combine immune response, which comprises treatment with the therapeutic combinations defined in the invention that may be simultaneously, separately or sequentially applied.

This invention is described in further details with the following procedures.

**Obtaining of an immunogenic preparation that contains mutated GnRH coupled to a Tetanic Toxoid T-helper epitope (D3-1), as one of the components for combined preparations.**

To fulfill antibody response against GnRH, a GnRH analogue peptide, conjugated to a carrier protein (mammal immunocastration vaccine, EPO 959079), is used for immunization. The GnRH analogue peptide (pGlu-His-Trp-Ser-Tyr-Pro-Leu-Arg-Pro-Gly), and a carrier protein (a Tetanic Toxoid T-helper epitope), were chemically synthesized using two glycine residues as separators, by solid phase method and Boc/Bzl strategy, using "4-methyl-benzhydrilamine" (MBH A-0.75 mmol/g, BACHEM, Swiss).

The humoral response against GnRH can be obtained through active immunization with natural GnRH or any of its analogues coupled to a carrier protein. Additionally, GnRH analogues, agonists or antagonists, may be used as such in combined preparations, with a synergistic effect in reducing the tumor mass, since they interrupt or cripple signaling through protein G in the cells that carry their receptors. Anti-GnRH antibodies can also be used as combined components to generate a passive immune response.

**Obtaining of immunogenic preparation containing recombinant human Epidermal Growth Factor (hrEGF) coupled to a carrier protein, as one component of combined preparations.**

A solution of recombinant human Epidermal Growth Factor (hrEGF) (National Medicament Register Office, Cuba, HEBERMIN, No. 1266) in PBS/MgCl$_2$ 10mM, is mixed with a carrier protein solution (recombinant P64, *Neisseria meningitides* outer membrane) in the same solvent; and a ration of 1:5 moles of hrEGF per protein mol. Late on, 0.05% of glutaraldehide is added to a final concentration of 0.05 to 0.1%. The mixture is incubated for 1-3 hours at room temperature and dialyzed in PBS/MgCl2 10 mM, with at least three changes of the dialysis solution (Vaccine composition comprising autologous epidermal growth factor or a fragment or a derivative thereof having anti-tumor activity and use thereof in the therapy of malignant diseases, US, 5894018).

Humoral response to EGF can also be achieved through EGF or its receptor's peptide immunization, coupled to an immunopotentiating carrier protein, passively or with direct administration of anti-EGF antibodies, or anti-EGF receptors.

EGF shares approximately 30% of its sequence with Transforming Growth Factor, TGF. They compete for the same binding sites of membrane receptors. Additionally, alpha TGF/EGF receptor complexes in different types of human tumors have been reported in large amounts. It is therefore evident, that the humoral response to TGF is important in the oncogenesis, and that is equally important in the sinergism that is described for the EGF

**Obtaining of an immunogenic preparation containing Human Vascular Endothelium Growth Factor (hVEGF), coupled to a carrier protein, as one of the components for combined preparations.**

Humoral response to VEGF is achieved by immunization using VEGF peptide conjugated to a carrier protein (KLH, keyhole limpet haemocyanin) (hVEGF-KLH). HVEGF$_{121}$ isoform conjugation to KLH was made with soluble carbodiimide coupling.

Humoral response to VEGF can also be achieved through immunization with VEGF peptides or its receptor, coupled to any immunopotentiating carrier protein, passively, or by direct administration of anti-VEGF receptors.

**Obtaining of a combined immunogenic preparation of GnRH and hrEGF.**

The combined immunogenic preparation was achieved by mixing 750 $\mu$g of D3-1 and 250 $\mu$g of hrEGF-P64 in a final volume of 0.5 ml.

**Obtaining of a combined immunogenic preparation of GnRH and hVEGF.**

The combined immunonegic formulation was achieved by mixing 750 $\mu$g of D3-1 and 100 $\mu$g of hVEGF-KLH in a final volume of 0.5 ml.

**Brief description of the drawings.**

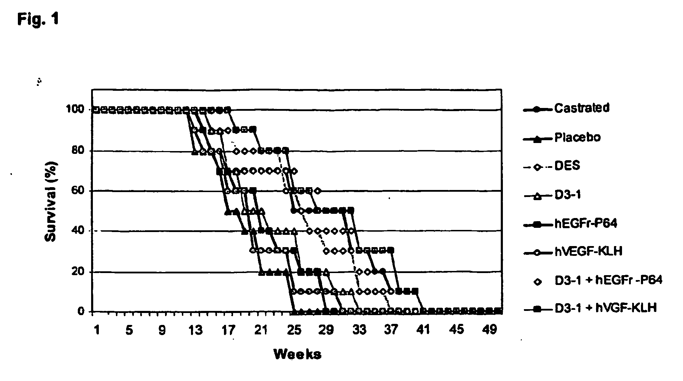

Figure 1 shows survival time evaluation of Copenhagen rats implanted with tumor line Dunning R 3327-G, subjected to different treatments.

**Detailed description of particular embodiments / Examples.**

This invention is illustrated with the following examples.

**1. Implant of tumor cell line R3327-G in Copenhagen rats.**

Tumor cell line Dunning R3327-G was implanted into 9-12 weeks old Copenhagen rats (with approximately 100 g of body weight each), which were subjected to different treatments with $2 \times 10^6$ cell density per animal in implant medium (RPMI 1640, serum-free in 0.5 ml), on the laxed area of the flanks. One hundred percent attachment efficiency was accomplished in the animals after 90 days.

**2. Evaluation of survival time, as anti-tumor activity criterion for rats implanted with tumor line Dunnning 3327-G under different treatments.**

Eight groups of ten animals each one, were formed for the experiment, using Copenhagen rats implanted as previously described.

Experimental groups:

1. Placebo animals (immunized with PBS in oily adjuvant).
2. Surgically castrated animals.
3. Animals treated with DES (dietilestilbestrol)
4. Animals immunized with peptide D3-1 (GnRHm1-TT).
5. Animals immunized with hrEGF-P64.
6. Animals immunized with hVEGF-KHL.
7. Animals immunized with a combined formulation of D3-1 + hrEGF-P64.
8. Animals immunized with a combined formulation of D3-1 + hVEGF-KLH.

The immunization scheme used for treatment included 7 doses (3 doses before the implant and 4 doses after) fortnightly administered: 750 $\mu$g of D3-1, 250 $\mu$g of hrEGF-P64, 100 $\mu$g of hrEGF-KLH, and combinations of D3-1 + hrEGF-P64 and D3-1 + hVEGF-KLH in a volume of 0.5 ml in oily adjuvant (Complete Freund Adjuvant was used in the first immunization, and Incomplete Freund Adjuvant was used in further stimulation), subcutaneously, on 4 sites on either side of the spine. The same antigen dose as that used for the independent treatment was kept in combined treatments.
DES treatment was made on and off, three times a week at a rate of 1 ml/kg/day, for as long as the experiment lasted, and started once the cells were inoculated.
Immunization began 30-45 days prior to the tumor implant procedure and lasted until 7 immunizations were completed; hence, humoral response in ELISA assay, expressed as antibody titers, was above the cut off value for each antigen, prior to cell inoculation.
The evaluation of the treatment effects was made once a week during the experimental period (13 months). The effect was evaluated as the animal survival time in each experimental group. The data are shown in figure 1.

**3. Evaluation of tumor reduction, as anti-tumor activity criterion in rats implanted with tumor cell line Dunning R3327-G under different treatments.**

Eight groups of ten animals each one were formed for the experiment, with Copenhagen rats as described before.

Experimental groups:

1. Placebo animals (immunized with PBS in oily adjuvant).
2. Surgically castrated animals.
3. Animals treated with DES (dietilestilbestrol)

4. Animals immunized with peptide D3-1 (GnRHm1-TT).

5. Animals immunized with hrEGF-P64.

6. Animals immunized with hVEGF-KHL.

7. Animals immunized with a combined formulation of D3-1 + hrEGF-P64.

8. Animals immunized with a combined formulation of D3-1 + hVEGF-KLH.

Like the previous, the immunization scheme used in the treatment included 7 doses (3 doses before the implant and 4 after), fortnightly administered: 750 $\mu$g of D3-1, 250 $\mu$g of hrEGF-P64, 100 $\mu$g of hVEGF-KLH and their combinations in 0.5 ml, in oily adjuvant (Complete Freund Adjuvant in the first immunization and Incomplete Freund Adjuvant in further stimulation), subcutaneously on 4 sites on either side of the spine.

DES treatment was made on and off, three times a week at a rate of 1mg/kg/day, as long as the experiment lasted; and it started once the cells were inoculated.

The evaluation of the experiment was made after sacrifice (three months following the tumor line implant). To evaluate the effect of each treatment, the tumor was dried and weighed in a technical balance. As treatment effect ($E_{treatment}$) was considered the unity, minus the mean weight ratio of tumors in the treated animals ($P_{treatment}$), and tumor mean weight in placebo animals ($P_{placebo}$):

$$E_{treatment} = 1 - (P_{treatment}/P_{placebo}). \qquad (1)$$

The expected effect ($E_{theoretical}$) in the case of double combination formulations can be calculated in case either effect is not mutually excluding (Caridad W., Guerra Bustillo, Ernesto Menendez Acuna, Rolando Barrera Morena, Esteban Egana Morales. "Estadistica", Editorial Pueblo y Educacion, 1989), according to:

$$E_{theoretical} = E_{T1} + E_{T2} - (E_{T1} {}^* E_{T2}). \qquad (2)$$

Where: $E_{T1}$ is treatment 1 effect, and $E_{T2}$ is treatment 2 effect.

As shown in table No.1, the experimental effect achieved for combinations (combined preparations) is higher than the expected theoretical effect, thus proving the synergic effect of these combinations in reducing tumor size.

**Table No.1. Effect of different treatments on Copenhagen rats implanted with the tumor cell line Dunning R3327-G at the time of sacrifice (three months following the tumor line implant).**

| Treatment | Tumor weight (g) (Mean of ten animals) | Treatment effect. | Theoretical effect. |
|---|---|---|---|
| Castrated | 9.20 | 0.707 | - |
| Placebo | 31.41 | 0.000 | - |
| DES | 12.66 | 0.597 | - |
| D3-1 | 19.31 | 0.385 | - |
| hrEGF-P64 | 25.43 | 0.190 | - |
| hVEGF-KLH | 21.72 | 0.309 | - |
| D3-1 + hEGFr-P64 | 12.14 | 0.613 | 0.502 |
| D3-1 + hVEGF-KLH | 10.87 | 0.654 | 0.575 |

## Claims

1. A pharmaceutical combination for the treatment of neoplasia through simultaneous or sequential administration, the combination comprising GnRH, optionally coupled to an immunopotentiating carrier protein, and a growth regulating factor selected from EGF and VEGF, optionally coupled to an immunopotentiating carrier protein.

**2.** A pharmaceutical combination according to claim 1, wherein said GnRH, EGF and/or VEGF is coupled to an immunopotentiating carrier protein by means of conjugation or the formation of chimeric proteins.

**3.** A pharmaceutical combination according to claim 1 or 2, which comprises a GnRH analogue peptide having the sequence pGlu-His-Trp-Ser-Tyr-Pro-Leu-Arg-Pro-Gly, coupled to an immunopotentiating carrier protein.

**4.** A pharmaceutical combination according to claim 1 or 2, wherein said immunopotentiating carrier protein is selected from Neisseria meningitides P1 and P64 outer membrane proteins.

**5.** A pharmaceutical combination according to claim 1 or 2, wherein said immunopotentiating carrier protein is a Tetanic Toxoid (TT) T helper epitope.

**6.** Use of GnRH, optionally coupled to an immunopotentiating carrier protein, for preparing a pharmaceutical composition for a treatment of neoplasia in combination with a growth regulating factor selected from EGF and VEGF, optionally coupled to an immunopotentiating carrier protein.

**7.** Use of a growth regulating factor selected from EGF and VEGF, optionally coupled to an immunopotentiating carrier protein, for preparing a pharmaceutical composition for a treatment of neoplasia in combination with GnRH, optionally coupled to an immunopotentiating carrier protein.

**8.** Use according to claim 6 or 7, wherein said treatment comprises simultaneous administration of said GnRH and said growth regulating factor.

**9.** Use according to claim 6 or 7, wherein said treatment comprises sequential administration of said GnRH and said growth regulating factor.

**Patentansprüche**

**1.** Pharmazeutische Kombination für die Behandlung von Neoplasie durch gleichzeitige oder aufeinanderfolgende Verabreichung, wobei die Kombination GnRH, das gegebenenfalls an ein immunpotenzierendes Trägerprotein gekoppelt ist, und einen Wachstum regulierenden Faktor, ausgewählt aus EGF und VEGF, der gegebenenfalls an ein immunpotenzierendes Trägerprotein gekoppelt ist, umfasst.

**2.** Pharmazeutische Kombination nach Anspruch 1, wobei das GnRH, EGF und/oder VEGF durch Konjugation oder die Bildung von chimären Proteinen an ein immunpotenzierendes Trägerprotein gekoppelt ist.

**3.** Pharmazeutische Kombination nach Anspruch 1 oder 2, welche ein GnRH-Analog-Peptid mit der Sequenz pGlu-His-Trp-Ser-Tyr-Pro-Leu-Arg-Pro-Gly, das an ein immunpotenzierendes Trägerprotein gekoppelt ist, umfasst.

**4.** Pharmazeutische Kombination nach Anspruch 1 oder 2, wobei das immunpotenzierende Trägerprotein ausgewählt ist aus Neisseria meningitides P1- und P64-Außenmembranproteinen.

**5.** Pharmazeutische Kombination nach Anspruch 1 oder 2, wobei das immunpotenzierende Trägerprotein ein Tetanustoxoid (TT)-T-Helfer-Epitop ist.

**6.** Verwendung von GnRH, das gegebenenfalls an ein immunpotenzierendes Trägerprotein gekoppelt ist, zum Herstellen einer pharmazeutischen Zusammensetzung für eine Behandlung von Neoplasie in Kombination mit einem Wachstum regulierenden Faktor, ausgewählt aus EGF und VEGF, der gegebenenfalls an ein immunpotenzierendes Trägerprotein gekoppelt ist.

**7.** Verwendung eines Wachstum regulierenden Faktors, ausgewählt aus EGF und VEGF, der gegebenenfalls an ein immunpotenzierendes Trägerprotein gekoppelt ist, zum Herstellen einer pharmazeutischen Zusammensetzung für eine Behandlung von Neoplasie in Kombination mit GnRH, das gegebenenfalls an ein immunpotenzierendes Trägerprotein gekoppelt ist.

**8.** Verwendung nach Anspruch 6 oder 7, wobei die Behandlung die gleichzeitige Verabreichung des GnRH und des wachstumsregulierenden Faktors umfasst.

9. Verwendung nach Anspruch 6 oder 7, wobei die Behandlung die aufeinanderfolgende Verabreichung des GnRH und des wachstumsregulierenden Faktors umfasst.


**Revendications**

1. Combinaison pharmaceutique pour le traitement des néoplasies par administration simultanée ou séquentielle, la combinaison comprenant GnRH, éventuellement couplée à une protéine support d'immunopotentialisation, et un facteur de régulation de croissance choisi parmi EGF et VGEF, éventuellement couplé à la protéine support d'immunopotentialisation.

2. Combinaison pharmaceutique selon la revendication 1, dans laquelle lesdits GnRH, EGF et/ou VGEF sont couplés à une protéine support d'immunopotentialisation au moyen d'une conjugaison ou de la formation de protéines chimères.

3. Combinaison pharmaceutique selon la revendication 1 ou 2, qui comprend un peptide analogue de GnRH ayant la séquence pGlu-His-Trp-Ser-Tyr-Pro-Leu-Arg-Pro-Gly, couplé à une protéine support d'immunopotentialisation.

4. Combinaison pharmaceutique selon la revendication 1 ou 2, dans laquelle ladite protéine support d'immunopotentialisation est choisie parmi les protéines P1 et les protéines membranaires externes P64 de Neisseria meningitidis.

5. Combinaison pharmaceutique selon la revendication 1 ou 2, dans laquelle ladite protéine support d'immunopotentialisation est un épitope des lymphocytes T auxiliaires de l'anatoxine tétanique (TT).

6. Utilisation de GnRH, éventuellement couplée à une protéine support d'immunopotentialisation, pour la préparation d'une composition pharmaceutique pour un traitement de néoplasies en combinaison avec un facteur de régulation de croissance choisi parmi EGF et VEGF, éventuellement couplé à une protéine support d'immunopotentialisation.

7. Utilisation d'un facteur de régulation de croissance choisi parmi EGF et VEGF, éventuellement couplé à une protéine de support d'immunopotentialisation, pour la préparation d'une composition pharmaceutique pour un traitement de néoplasies en combinaison avec GnRH, éventuellement couplée à une protéine support d'immunopotentialisation.

8. Utilisation selon la revendication 6 ou 7, dans laquelle ledit traitement comprend l'administration simultanée de ladite GnRH et dudit facteur de régulation de croissance.

9. Utilisation selon la revendication 6 ou 7, dans laquelle ledit traitement comprend l'administration séquentielle de ladite GnRH et dudit facteur de régulation de croissance.

**Fig. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5894018 A

**Non-patent literature cited in the description**

- **Jennes L ; Conn P.M.** Gonodatripin-releasing hormone and its receptors in the rat brain. *Front Neuroendocrinol,* 1994, vol. 15, 51-77
- **Peng C. ; Fan N.C. ; Ligier M. ; Vaananen J. ; Leung P.C.** Expression and regulation of gonadotropin-releasing hormone (GnRH) and GnRH receptor messenger ribonucleic acids in human granulosa-luteal cells. *Endocrinology,* 1994, vol. 135, 1740-1746
- **Di Matteo L. ; Vallarino M. ; Pierantoni R.** Localization of GnRH molecular forms in the brain, pituitary and testis of the frog, Rana esculenta. *J. Exp. Zool.,* 1996, vol. 247, 33-40
- **Gohar J. ; Mazor M. ; Lieberman J.R.** GnRH in pregnancy. *Arch. Gynecol. Obstet.,* 1996, vol. 259, 1-6
- **Jacobson J. D. ; Crofford L.J. ; Sun L. ; Wilder R. L.** Cyclical expression of GnRH and GnRH receptor mRNA in lymphoid organs. *Neuroendocrinology,* 1998, vol. 67, 117-125
- **Bauer T.W. ; Moriarty C.M. ; Childs G. V.** Studies of immunoreactive gonadotropin releasing hormone (GnRH) in the rat anterior pituitary. *J. Histochem. Cytochem,* 1981, vol. 29, 1171-1178
- **Couillard S. ; Labrie C. ; Belanger A. ; Candas B. ; Pouliot F. ; Labrie F.** Effect of dehydroepiandrosterone and anti-androgen EM-800 on growth of human ZR-75-1 breast cancer xenografts. *J. Nat. Cancer Inst.,* 20 May 1998, 772-778
- **Kolle S. et al.** Expression of growth hormone receptor in human prostatic carcinoma and hyperplasia. *Int. J. Oncol.,* 1999, vol. 14 (5), 911-916
- **A Jungwirth et al.** Inhibition of in vivo proliferation of androgen-independent prostate cancers by an antagonist of growth hormone-releasing hormone. *British Journal of Cancer,* 1997, vol. 75 (11), 1585-1592
- **Damber J.E. ; Bergh B. ; Gafvels M.** Epidermal growth factor receptor content in rat prostatic adenocarcinoma: effects of endocrine treatment. *Urol. Res.,* 1995, vol. 23 (2), 119-25

- Coordinate loss of growth factors following castration of rats carrying the Dunning R3327 G prostatic tumor. *Clin Physiol Biochem,* 1992, vol. 9 (2), 47-50
- **Cohen S. ; Carpenter G.** Human Epidermal Growth Factor: Isolation and chemical and biological properties. *PNAS USA,* 1975, vol. 72, 1317
- **Cohen S.** *J. Biol. Chem.,* 1962, vol. 237 (1), 555
- **Cohen S.** Human Epidermal Growth Factor: Isolation and Chemical and Biological Protperties. *PNAS USA,* 1975, vol. 72, 1317
- **Helding C.H.** *Cell,* 1984, vol. 37, 9-20
- **Rios M.A. et al.** Receptors for Epidermal Growth Factor and Estrogen Predictors of Relapse in Patients with Mammary Carcinoma. *Anticancer Research,* 1998, vol. 8, 173-176
- **Perez R. ; Pascual M.R. ; Macias A. ; Lage A.** *Breast Cancer Research and Treatment,* 1984, vol. 4, 189-193
- **Lombardero J. et al.** *Neoplasma,* 1987, vol. 33, 4
- Expression of vascular endothelial growth factor and its receptors in the ventral prostate and Dunning R3327 PAP adenocarcinoma before and after castration. *Prostate,* 1998, vol. 36 (2), 71-79
- **Laura E. et al.** Selective ablation of immature blood vessels in established human tumors follows vascular endothelial growth factor withdrawal. *J. Clin. Invest.,* 1999, vol. 103 (2), 159-165
- **Yves A. Muller et al.** The crystal structure of vascular endothelial growth factor (VEGF) refined to 1.93 A resolution: Multiple copy flexibility and receptor binding. *Structure,* 1997, vol. 5 (10), 1325-1338
- **Douglas H. ; Robert A. W.** The Hallmarks of Cancer (Review). *Cell,* 2000, vol. 100, 57-70
- **Caridad W. ; Guerra Bustillo ; Ernesto Menendez Acuna ; Rolando Barrera Morena ; Esteban Egana Morales.** Estadistica. *Editorial Pueblo y Educacion,* 1989